# EUROPEAN PATENT APPLICATION

(11) **EP 4 008 822 A1**
(43) Date of publication of application: **08.06.2022**
(21) Application number: 20860178.1
(22) Date of filing: 03.09.2020
(51) Int. Cl.: D04H 1/4266, A61F 13/00, A61L 15/32, A61L 27/36, A61L 27/50, C12M 1/00, C12M 3/00, D01F 4/02, D04H 1/728

(54) **SILK FIBER-CONTAINING NONWOVEN FABRIC, WOUND DRESSING, IPS CELL SCAFFOLD MATERIAL, NONWOVEN FABRIC FOR BLOOD COMPATIBLE MATERIAL, BLOOD COMPATIBLE MATERIAL, METHOD FOR PRODUCING SILK FIBER-CONTAINING NONWOVEN FABRIC, METHOD FOR PRODUCING WOUND DRESSING, METHOD FOR PRODUCING IPS CELL SCAFFOLD MATERIAL, METHOD FOR PRODUCING NONWOVEN FABRIC FOR BLOOD COMPATIBLE MATERIAL, AND METHOD FOR PRODUCING BLOOD COMPATIBLE MATERIAL**

(30) Priority: 06.09.2019 JP 2019163170; 06.09.2019 JP 2019163172; 08.10.2019 JP 2019185461; 08.10.2019 JP 2019185462
(71) Applicant: Central Glass Co., Ltd., Yamaguchi 755-0001 (JP)
(72) Inventor: SEKIYA, Yuki, Kawagoe-shi, Saitama 350-1159 (JP); KAWAI, Wataru, Kawagoe-shi, Saitama 350-1159 (JP); HAGIWARA, Yuki, Kawagoe-shi, Saitama 350-1159 (JP); MATSUNAGA, Kei, Kawagoe-shi, Saitama 350-1159 (JP)
(74) Representative: Manitz Finsterwald Patent- und Rechtsanwaltspartnerschaft mbB
(86) International application number: PCT/JP2020/033488
(87) International publication number: WO 2021/045167

(57) **Abstract**

One embodiment of the present invention provides a nonwoven fabric containing silk fibers in which an abs intensity ratio [abs (1650)/abs (1620)], which is a ratio of an intensity of a peak positioned in a vicinity of 1650 cm⁻¹ [abs (1650)] in an infrared absorption spectrum to an intensity of a peak positioned in a vicinity of 1620 cm⁻¹ [abs (1620)] in an infrared absorption spectrum, is larger than 0.65 and 1.90 or less, and a method for producing the nonwoven fabric containing silk fibers.

## Description

### TECHNICAL FIELD

One embodiment of the present invention relates to a nonwoven fabric containing silk fibers, a wound dressing, an iPS cell scaffold material, a nonwoven fabric for a blood-compatible material, a blood-compatible material, a production method of the nonwoven fabric containing the silk fibers, a production method of the wound dressing, a production method of the iPS cell scaffold material, a production method of the nonwoven fabric for the blood-compatible material, and a production method of the blood-compatible material.

### BACKGROUND ART

Patent Literature 1 discloses an undecomposed silk fibroin obtained by refining a cocoon layer or a cocoon filament of raw cocoons, dry cocoons, or boiled cocoons, a raw silk, a silk fabric, or a residual yarn thereof.

Patent Literature 2 states that a fibroin molded body produced from silk fibroin obtained by the method described in Patent Literature 1 exhibits high water solubility, and there is a disadvantage that the fibroin molded body is dissolved or weakened in strength by contact with water.

Patent Literature 2 further states that a treatment for the purpose of insolubilization (for example, induction of crystallization caused by alcohol or induction of crystallization caused by heat treatment) is known in order to overcome this disadvantage.

Patent Literature 3 discloses that a tubular structure is formed from a silk fibroin solution by an electrospinning method, and the tubular structure is coated with a silk fibroin sponge so as to be used for an artificial blood vessel requiring blood compatibility.

Patent Literature 4 discloses a fibrous membrane for tissue repair, which is formed by entangling fiber yarns having a diameter of 10 nm to 100 µm, has a porous structure, has a bulkiness of 200 to 2,000 cm³/g, and has a flexibility of 50 to 500 mN.

Patent Literature 5 discloses a cell scaffold material for osteoblasts or the like, which is formed of silk composite nanofibers that are made of silk and polyoxyic acid in an optional ratio and have a fiber diameter of 100 nm to 1,000 nm.

### CITATION LIST

### PATENT LITERATURE

Patent Literature 1: JP-A-2001-163899
Patent Literature 2: JP-A-2017-149674
Patent Literature 3: JP-A-2010-137041
Patent Literature 4: Japanese Patent No. 6140295
Patent Literature 5: JP-A-2014-015702

### SUMMARY OF INVENTION

### TECHNICAL PROBLEM

However, in a case where the insolubilization treatment with an alcohol as described in Patent Literature 2 is performed, toughness may be lowered (incentives for brittleness), and there is room for further improvement from the viewpoint of water resistance and toughness.

Further improvement is required for the artificial blood vessel from the viewpoint of water resistance and toughness, which is not limited to the case described in Patent Literature 3 in which the tubular structure is formed from a silk fibroin solution by an electrospinning method, and the tubular structure is coated with silk fibroin sponge so as to be used for an artificial blood vessel.

A blood-compatible material such as an artificial blood vessel is also required to prevent leakage of blood and adhesion of blood.

There is room for improvement in the healing effect of the fibrous membrane for tissue repair described in Patent Literature 4.

A wound dressing is known as a material for covering a wound on the skin, and the wound dressing is required to have water resistance for use on the premise of contact with the blood or body fluids from skin wounds and is further required to have high toughness in view of followability after application to a damaged site and durability to deformation such as bending and stretching of the skin.

A scaffold material, which enables iPS cells that have been actively studied in recent years and are not limited to osteoblasts described in Patent Literature 5 to form a three-dimensional tissue structure during proliferation, has been required.

In view of the fact that a culture solution for culturing iPS cells is generally water, the iPS cell scaffold material needs to have water resistance. Furthermore, in view of processing and use of the scaffold material, the iPS cell scaffold material needs to have good handleability, in other words, high toughness.

An aspect of the present invention has been made in view of the above problems, and an object thereof is to provide a nonwoven fabric containing silk fibers, which has high water resistance and high toughness, and a method for producing the nonwoven fabric.

Another aspect of the present invention has been made in view of the above problems, and an object thereof is to provide a nonwoven fabric for a blood-compatible material and a blood-compatible material which have high water resistance and high toughness and can prevent leakage of blood and adhesion of blood, a method for producing the nonwoven fabric for the blood-compatible material, and a method for producing the blood-compatible material.

Still another aspect of the present invention has been made in view of the above problems, and an object thereof is to provide a wound dressing that has high water resistance, high toughness, and excellent healing effects, and a method for producing the wound dressing.

Yet still another aspect of the present invention has been made in view of the above problems, and an object thereof is to provide an iPS cell scaffold material that has high water resistance and high toughness and enables iPS cells to form a three-dimensional tissue structure during proliferation, and a method for producing the iPS cell scaffold material.

### SOLUTION TO PROBLEM

The present inventors have made intensive studies and have found that a nonwoven fabric containing silk fibers has high water resistance and high toughness, in which an abs intensity ratio [abs (1650)/abs (1620)], which is a ratio of an intensity of a peak positioned in a vicinity of 1650 cm⁻¹ [abs (1650)] in an infrared absorption spectrum to an intensity of a peak positioned in a vicinity of 1620 cm⁻¹ [abs (1620)] in an infrared absorption spectrum, is larger than 0.65 and 1.90 or less.

The present inventors have found that a nonwoven fabric for a blood-compatible material, which has high water resistance and high toughness and can prevent leakage of blood and adhesion of blood, can be obtained by the above nonwoven fabric.

In addition, the present inventors have found that a wound dressing having high water resistance, high toughness, and excellent healing effects can be obtained by using the nonwoven fabric.

Furthermore, the present inventors have found that, by using the nonwoven fabric, an iPS cell scaffold material that has high water resistance and high toughness and enables iPS cells to form a three-dimensional tissue structure during proliferation can be obtained.

The present invention includes the following aspects.
<1> A nonwoven fabric containing silk fibers, in which an abs intensity ratio [abs (1650)/abs (1620)], which is a ratio of an intensity of a peak positioned in a vicinity of 1650 cm⁻¹ [abs (1650)] in an infrared absorption spectrum to an intensity of a peak positioned in a vicinity of 1620 cm⁻¹ [abs (1620)] in an infrared absorption spectrum, is larger than 0.65 and 1.90 or less.
<2> The nonwoven fabric containing silk fibers according to <1>, in which the abs intensity ratio [abs (1650)/abs (1620)] is 0.70 or more and 1.00 or less.
<3> The nonwoven fabric containing silk fibers according to <1> or <2>, in which the nonwoven fabric is a nonwoven fabric for a blood compatible material.
<4> A blood compatible material including the nonwoven fabric containing silk fibers according to <3>.
<5> The blood compatible material according to <4>, in which the blood compatible material is an artificial blood vessel, a catheter, a blood filter, an anti-adhesion material, a stent, a cell scaffold material, an artificial organ, a blood storage container, or a blood transfusion equipment.
<6> The nonwoven fabric containing silk fibers according to <1> or <2>, in which the nonwoven fabric is a wound dressing.
<7> The nonwoven fabric containing silk fibers according to <1> or <2>, in which the nonwoven fabric is an iPS cell scaffold material.
<8> A method for producing a nonwoven fabric containing silk fibers including:
   dissolving silk fibroin in a fluorine-based alcohol to obtain a silk fibroin solution; and
   performing electrospinning using the silk fibroin solution,
   in which an abs intensity ratio [abs (1650)/abs (1620)] of the silk fibers, which is a ratio of an intensity of a peak positioned in a vicinity of 1650 cm⁻¹ [abs (1650)] in an infrared absorption spectrum to an intensity of a peak positioned in a vicinity of 1620 cm⁻¹ [abs (1620)] in an infrared absorption spectrum, is larger than 0.65 and 1.90 or less.
<9> The method for producing a nonwoven fabric containing silk fibers according to <8>, in which the fluorine-based alcohol is a solvent containing 70 mass% or more of 1,1,1,3,3,3-hexafluoro-2-propanol.
<10> The method for producing a nonwoven fabric containing silk fibers according to <8> or <9>, in which the silk fibroin solution is obtained by dissolving silk fibroin in the solvent containing 70 mass% or more of 1,1,1,3,3,3-hexafluoro-2-propanol at a dissolution temperature of 58.6°C or higher and 180°C or lower.
<11> The method for producing a nonwoven fabric containing silk fibers according to any one of <8> to <10>, in which the fluorine-based alcohol is 1,1,1,3,3,3-hexafluoro-2-propanol.
<12> The method for producing a nonwoven fabric containing silk fibers according to any one of <8> to <11>, further including a step of bringing the nonwoven fabric containing silk fibers obtained by electrospinning into contact with an alcohol.
<13> The method for producing a nonwoven fabric containing silk fibers according to <12>, in which the alcohol is ethanol or an aqueous solution thereof.
<14> The method for producing a nonwoven fabric containing silk fibers according to any one of <8> to <13>, in which the nonwoven fabric is a wound dressing.
<15> The method for producing a nonwoven fabric containing silk fibers according to any one of <8> to <13>, in which the nonwoven fabric is an iPS cell scaffold material.
<16> The method for producing a nonwoven fabric containing silk fibers according to any one of <8> to <13>, in which the nonwoven fabric is a nonwoven fabric for a blood compatible material.
<17> A method for producing a blood compatible material, including the method for producing a nonwoven fabric containing silk fibers according to <16>.

### ADVANTAGEOUS EFFECTS OF INVENTION

According to an aspect of the present invention, it is possible to provide a nonwoven fabric containing silk fibers, which has high water resistance and high toughness, and a method for producing the nonwoven fabric.

According to another aspect of the present invention, it is possible to provide a nonwoven fabric for a blood-compatible material and a blood-compatible material which have high water resistance and high toughness and can prevent leakage of blood and adhesion of blood, a method for producing the nonwoven fabric for the blood-compatible material, and a method for producing the blood-compatible material.

According to still another aspect of the present invention, it is possible to provide a wound dressing that has high water resistance, high toughness, and excellent healing effects, and a method for producing the wound dressing.

According to yet still another aspect of the present invention, it is possible to provide an iPS cell scaffold material that has high water resistance and high toughness and enables iPS cells to form a three-dimensional tissue structure during proliferation, and a method for producing the iPS cell scaffold material.

### BRIEF DESCRIPTION OF DRAWINGS

FIG. 1 is a microscopic observation photograph showing a state of iPS cells in Examples during proliferation, which is obtained by performing microscope observation after respective cultures of the first passage, the second passage, and the third passage in a culture test of iPS cells.
FIG. 2 is a microscopic observation photograph showing a state of iPS cells in Comparative Examples during proliferation, which is obtained by performing microscope observation after respective cultures of the first passage, the second passage, and the third passage in a culture test of iPS cells.

### DESCRIPTION OF EMBODIMENTS

A nonwoven fabric containing silk fibers according to an embodiment of the present invention is a nonwoven fabric containing silk fibers in which an abs intensity ratio [abs (1650)/abs (1620)], which is a ratio of an intensity of a peak positioned in a vicinity of 1650 cm⁻¹ [abs (1650)] in an infrared absorption spectrum to an intensity of a peak positioned in a vicinity of 1620 cm⁻¹ [abs (1620)] in an infrared absorption spectrum, is larger than 0.65 and 1.90 or less .

The peak intensity [abs (1650)] is derived from an α-helix structure of the silk fibers, and the peak intensity [abs (1620)] is derived from a β-sheet structure of the silk fiber.

In the present specification, the vicinity of 1650 cm⁻¹ in the infrared absorption spectrum means a range of ± 10 cm⁻¹ around 1650 cm⁻¹, and the peak positioned in the vicinity of 1650 cm⁻¹ in the infrared absorption spectrum means a maximum peak within a range of ±10 cm⁻¹ around 1650 cm⁻¹.

In the present specification, the vicinity of 1620 cm⁻¹ in the infrared absorption spectrum means a range of ±10 cm⁻¹ around 1620 cm⁻¹, and the peak positioned in the vicinity of 1620 cm⁻¹ in the infrared absorption spectrum means a maximum peak within a range of ±10 cm⁻¹ around 1620 cm⁻¹.

In the nonwoven fabric containing silk fibers according to an embodiment of the present invention, the abs intensity ratio [abs (1650)/abs (1620)] of the silk fibers contained in the nonwoven fabric is larger than 0.65 and 1.90 or less, so that high water resistance and high toughness can be achieved.

In a nonwoven fabric for a blood-compatible material according to another embodiment of the present invention, the abs intensity ratio [abs (1650)/abs (1620)] of the silk fibers contained in the nonwoven fabric for the blood-compatible material is larger than 0.65 and 1.90 or less, so that high water resistance, high toughness, prevention of leakage of blood, and prevention of adhesion of blood can be achieved.

In a wound dressing according to still another embodiment of the present invention, the nonwoven fabric containing silk fibers having an abs intensity ratio [abs (1650)/abs (1620)] of larger than 0.65 and 1.90 or less is used, so that high water resistance, high toughness, and excellent healing effects can be achieved.

Furthermore, in an iPS cell scaffold material according to yet still another embodiment of the present invention, the nonwoven fabric containing silk fibers having an abs intensity ratio [abs (1650)/abs (1620)] of larger than 0.65 and 1.90 or less is used, so that water resistance is high, toughness is high, and iPS cells can form a three-dimensional tissue structure during proliferation.

Although the details are not clear, it is presumed as follows.

It is considered that the brittleness of the nonwoven fabric can be improved by setting the abs intensity ratio [abs (1650)/abs (1620)] to be larger than 0.65. It is considered that the toughness based on the β-sheet structure is ensured when the peak intensity [abs (1620)] is large, and the toughness is increased due to the α-helix structure since the peak intensity [abs (1650)] is not too low.

It is considered that high water resistance can be obtained by setting the abs intensity ratio [abs (1650)/abs (1620)] to be 1.90 or less. It is considered that the strong toughness based on the β-sheet structure can be ensured when the peak intensity [abs (1620)] is present, and since the peak intensity [abs (1650)] is not too high, the dissolution in water due to the α-helix structure can be prevented, the shape is easily maintained against water, and water resistance is ensured.

Furthermore, it is considered that silk fibroin is commonly known to have excellent biocompatibility, and the use of a nonwoven fabric for a blood compatible material, which is a nonwoven fabric containing silk fibers, can prevent the leakage of blood and the adhesion of blood.

It is presumed that the silk fibers in the nonwoven fabric for the blood compatible material according to the other embodiment of the present invention are generally obtained from silk fibroin, and most of the secondary structures thereof is not an α-helix helical structure but a β-sheet planar structure, and therefore, the silk fibers are a water-insoluble and tough material, and as a blood-compatible material, the silk fibers prevent the adhesion of blood while preventing leakage of blood due to physical external stimuli.

As described above, it is considered that the brittleness of the nonwoven fabric can be improved by setting the abs intensity ratio [abs (1650)/abs (1620)] to be larger than 0.65. It is considered that the strong toughness based on the β-sheet structure can be ensured when the peak intensity [abs (1620)] is large, and since the peak intensity [abs (1650)] is not too low, the water compatibility of the wound dressing due to the α-helix structure can also be ensured, and the toughness is increased, in other words, the shape of the nonwoven fabric can be maintained.

It is considered that high water resistance can be obtained by setting the abs intensity ratio [abs (1650)/abs (1620)] to be 1.90 or less. It is considered that the strong toughness based on the β-sheet structure can be ensured when the peak intensity [abs (1620)] is present, and since the peak intensity [abs (1650)] is not too high, the dissolution of the wound dressing in water due to the α-helix structure can be prevented, the shape is easily maintained against water, and water resistance is ensured.

Furthermore, it is considered that an excellent healing effect can be obtained by using a nonwoven fabric containing silk fibers as a wound dressing.

Silk fibroin derived from a silk worm is said to be a material having excellent biocompatibility, and has also been used for, for example, surgical suture threads (trade name: Mani Silk, manufactured by MANI, INC.).

It is presumed that the silk fibers in the wound dressing according to still another embodiment of the present invention is generally obtained from silk fibroin, and most of the secondary structures of the silk fibers in the nonwoven fabric according to one embodiment of the present invention is not an α-helix helical structure but a β-sheet planar structure, and therefore, the silk fibers are a water-insoluble and tough material, and as a wound dressing, the silk fibers protect the wound from physical external stimuli and are improved in the healing effect.

In general, in the wound healing process, epithelial tissue of the skin is finally regenerated through stages of a hemostatic phase, an inflammatory phase, a cell proliferation phase, and a maturation phase, and it is considered that the nonwoven fabric containing silk fibers is used as a wound dressing to contribute to tissue regeneration (particularly regeneration of the epithelial layer) during these healing processes.

It is considered that the brittleness of the nonwoven fabric can be improved and the nonwoven fabric can be used as an iPS cell scaffold material by setting the abs intensity ratio [abs (1650)/abs (1620)] to be larger than 0.65. Although the reason for this is not clear, it is considered that the strong toughness based on the β-sheet structure can be ensured when the peak intensity [abs (1620)] is large, and since the peak intensity [abs (1650)] is not too low, the water compatibility of the iPS cell scaffold material due to the α-helix structure can also be ensured, and the toughness is increased, in other words, the shape of the nonwoven fabric can be maintained.

It is considered that high water resistance can be obtained by setting the abs intensity ratio [abs (1650)/abs (1620)] to be 1.90 or less. Although the reason for this is not clear, it is considered that the strong toughness based on the β-sheet structure can be ensured when the peak intensity [abs (1620)] is present, and since the peak intensity [abs (1650)] is not too high, the dissolution of the iPS cell scaffold material in water due to the α-helix structure can be prevented, the shape is easily maintained against water, and water resistance is ensured.

Furthermore, it is considered that the iPS cells can form a three-dimensional tissue structure during proliferation by using a nonwoven fabric containing silk fibers as the iPS cell scaffold material.

Silk fibroin derived from a silk worm is said to be a material having excellent biocompatibility, and has also been used for, for example, surgical suture threads (brand name: Mani Silk, manufactured by MANI, INC.).

It is presumed that the silk fibers in the present invention is generally obtained from silk fibroin, and most of the secondary structures of the silk fibers in the nonwoven fabric according to one embodiment of the present invention is not an α-helix helical structure but a β-sheet planar structure, and therefore, the silk fibers are a water-insoluble and tough material, and can function as a scaffolding material that is insoluble in the medium during cell culture although the exact reason is unknown.

It is presumed that the nonwoven fabric containing silk fibers is used as the iPS cell scaffold material to exhibit an appropriate biocompatibility with the iPS cells, and therefore, the behavior of the cell proliferation does not proceed in a planar manner but proceeds in a three-dimensional manner with respect to the scaffold material although details are unknown.

The abs intensity ratio is larger than 0.65 and 1.90 or less, preferably 0.70 or more and 1.00 or less, and more preferably 0.70 or more and 0.80 or less.

When the abs intensity ratio is 0.65 or less, toughness cannot be secured. On the other hand, when the abs intensity ratio is larger than 1.90, water resistance cannot be ensured.

A method for adjusting the abs intensity ratio to be larger than 0.65 and 1.90 or less is not particularly limited, and examples thereof include a method in which silk fibroin as a raw material is dissolved in a fluorine-based alcohol to obtain a silk fibroin solution, and the silk fibroin solution subjected to electrospinning to obtain a nonwoven fabric containing silk fibers.

The above nonwoven fabric containing silk fibers (also simply referred to as "nonwoven fabric") is preferably a nonwoven fabric for a blood compatible material.

The above nonwoven fabric is preferably a wound dressing.

The nonwoven fabric is preferably an iPS cell scaffold material.

The silk fibroin used as a raw material can be obtained by excluding impurities from silkworm cocoon balls or raw silk, in the nonwoven fabric containing silk fibers according to one embodiment of the present invention and a production method thereof, the nonwoven fabric for the blood compatible material according to another embodiment of the present invention and a production method thereof, the wound dressing according to still another embodiment of the present invention and a production method thereof, and the iPS cell scaffold material according to yet still another embodiment of the present invention and a production method thereof. The cocoon filaments spit out by larvae of silkworms are composed of filaments of fibroin and agglutinous sericin covering the filaments, and the cocoon balls are produced by adhering the filaments of fibroin with sericin.

The content of fibroin in the cocoon balls is 70 mass% or more and 80 mass% or less, and the content of sericin is 20 mass% or more and 30 mass% or less. A small amount of other impurities such as a fat is contained. The raw silk is taken out from cocoons after the cocoon balls are immersed and disentangled in hot water, and contains impurities.

The species of silkworms for producing silk fibroin, which is used for the nonwoven fabric containing silk fibers according to one embodiment of the present invention and a production method thereof, the nonwoven fabric for the blood compatible material according to another embodiment of the present invention and a production method thereof, the wound dressing according to still another embodiment of the present invention and a production method thereof, and the iPS cell scaffold material according to yet still another embodiment of the present invention and a production method thereof, are not particularly limited, and examples thereof include wild silkworms such as eri-silkworms, antheraea pernyi, and Japanese oak silkmoth, or domestic silkworms.

For example, the silk fibroin obtained through refining can be used in the nonwoven fabric containing silk fibers according to one embodiment of the present invention and a production method thereof, the nonwoven fabric for the blood compatible material according to another embodiment of the present invention and a production method thereof, the wound dressing according to still another embodiment of the present invention and a production method thereof, and the iPS cell scaffold material according to yet still another embodiment of the present invention and a production method thereof. The refining is a step of removing the impurities from the cocoon balls, and for example, the impurities can be removed by bringing the cocoon balls or raw silks into contact with an alkaline solution such as a soap solution, lye, or a soda solution. Silk fibroin that is commercially available as silk yarns, silk woven fabrics, health food, or the like may also be used. Silk fibroin that is directly collected from a silk gland of larvae of silkworms may also be used.

There is no particular limitation on the molecular weight (Mw) of the silk fibroin used for the nonwoven fabric containing silk fibers according to one embodiment of the present invention and a production method thereof, the nonwoven fabric for the blood compatible material according to another embodiment of the present invention and a production method thereof, the wound dressing according to still another embodiment of the present invention and a production method thereof, and the iPS cell scaffold material according to yet still another embodiment of the present invention and a production method thereof. The Mw of the silk fibroin is generally 50,000 to 370,000, although it depends on the type of the alkaline solution used in the refining of cocoon balls or raw silks and the refining conditions. The literature value of Mw of silk fibroin produced from silk glands of domestic silkworms is 370,000 (described in Non-Patent Literature: Tashiro Yutaka and Otsuki Eiichi, Journal of Cell Biology, Vol.46, P1(1970), Non-Patent Literature: Prog. Polym.Sci.2007; 32 (8-9): 991-1007).

The fluorine-based alcohol (also referred to as "fluoroalcohol") is not particularly limited as long as it can dissolve silk fibroin, and examples thereof include fluoroalcohols such as 1,1,1,3,3,3-hexafluoro-2-propanol (HFIP).

The fluorine-based alcohol is preferably HFIP. The fluoroalcohols may be used alone or in combination. Further, the fluoroalcohol may be used by being mixed with one kind or two or more kinds of other solvents. As the other solvents, any solvent may be used as long as it does not cause deterioration of silk fibroin and a decrease in molecular weight of silk fibroin and does not hinder dissolution. Examples of such a solvent include N-methylmorpholine N-oxide, 2,2,2-trifluoroethanol, methanol, acetone, tetrahydrofuran, N,N-dimethylacetamide, toluene, and dichloromethane. These solvents can be used within the range of 30 or less (preferably 10 or less) in terms of a relative ratio with respect to the mass of the fluoroalcohol as 100 when the silk fibroin solution is obtained.

In a preferred embodiment, the fluorine-based alcohol is a solvent containing 70 mass% or more of 1,1,1,3,3,3-hexafluoro-2-propanol.

The silk fibroin solution is obtained by dissolving silk fibroin as a raw material in a fluorine-based alcohol.

The container used when silk fibroin is dissolved in a fluorine-based alcohol is not particularly limited as long as it has airtightness and pressure resistance. For example, a sealed pressure-resistant container made of glass, stainless steel, or obtained by lining the glass or the stainless steel with a resin can be used.

In a preferred embodiment, the silk fibroin solution is obtained by dissolving silk fibroin in a solvent containing 70 mass% or more of 1,1,1,3,3,3-hexafluoro-2-propanol. The dissolution temperature at which silk fibroin is dissolved in a solvent containing 70 mass% or more of 1,1,1,3,3,3-hexafluoro-2-propanol is not particularly limited, and is preferably 58.6°C or higher and 180°C or lower.

The dissolution temperature being 58.6°C or more, that is the boiling point of HFIP at atmospheric pressure (1013.25 hPa), is preferred since the dissolution rate of silk fibroin does not decrease, and the recovered amount of insoluble matter does not increase. The dissolution temperature is preferably 60°C or higher. The dissolution temperature being 180°C or lower is preferred since the solution is less likely to be colored due to hydrolysis or alteration of silk fibroin. The dissolution temperature is preferably 140°C or lower, and particularly preferably 120°C or lower.

In a preferred embodiment, the dissolution time of silk fibroin in a solvent containing 70% by mass or more of 1,1,1,3,3,3-hexafluoro-2-propanol is 0.5 hours or more and 24 hours or less. The dissolution time being 0.5 hours or more is preferred since the silk fibroin is sufficiently dissolved, and the recovered amount of insoluble matter is not easily increased. The dissolution time being 24 hours or less is preferred since productivity is high and practical. In addition, the dissolution time being 24 hours or less is preferred since silk fibroin is not easily hydrolyzed to cause a decrease in the molecular weight, and the HFIP solution of silk fibroin is not easily colored. The dissolution time is preferably within 12 hours, and particularly preferably within 6 hours. In order to avoid unnecessary heating, it is preferable that the progress of dissolution is appropriately checked, and after the dissolution is completed, the solution is rapidly cooled to end the dissolution operation. At this time, stirring may be performed for the purpose of shortening the dissolution time.

The concentration of the silk fibroin in the silk fibroin solution is preferably 0.01 mass% or more and 50 mass% or less relative to the total silk fibroin solution. The concentration of the silk fibroin is more preferably 0.1 mass% or more and 30 mass% or less, still more preferably 1 mass% or more and 10 mass% or less, and particularly preferably 1 mass% or more and 5 mass% or less.

When the concentration is 0.01 mass% or more, the productivity of the obtained nonwoven fabric containing silk fibers is easily ensured, and when the concentration is 50 mass% or less, the viscosity of the solution is not excessively high, and thus the nonwoven fabric is easily molded.

When the concentration is 0.01 mass% or more, the productivity of the obtained nonwoven fabric for a blood compatible material is easily ensured, and when the concentration is 50 mass% or less, the viscosity of the solution is not excessively high, and thus the nonwoven fabric for a blood compatible material is easily molded.

When the concentration is 0.01 mass% or more, the productivity of the obtained wound dressing is easily ensured, and when the concentration is 50 mass% or less, the viscosity of the solution is not excessively high, and thus the wound dressing is easily molded.

The concentration being 0.01 mass% or more is preferred since the productivity of the obtained iPS cell scaffold material can be ensured, and the concentration being 50 mass% or less is preferred since the viscosity of the silk fibroin solution is not excessively high, and the iPS cell scaffold material is easily molded.

The method for producing a silk fibroin solution according to one embodiment of the present invention can be performed in an inert gas atmosphere such as nitrogen gas or helium gas, or in an air atmosphere, and is preferably performed in an inert gas atmosphere. The air atmosphere may promote hydrolysis and coloring of the solution.

The concentration of the silk fibroin solution will be described below.

After preparing a dilute silk fibroin solution, the solution is concentrated by distilling off a part of the fluorine-based alcohol in the solution, so that a high concentration silk fibroin solution can be obtained. For example, after preparing an HFIP solution of silk fibroin having a concentration of 1 mass%, an HFIP solution of silk fibroin having a concentration of 10 mass% or more can be obtained using an evaporator. Although the used amount of HFIP increases, an effect of shortening the dissolution time is exhibited.

Examples of a method for obtaining a nonwoven fabric containing silk fibers by using a silk fibroin solution include an electrospinning method.

The electrospinning method is a method in which a high voltage is applied between a nozzle (needle) and an electrode (collector), and in this state, a polymer solution is sprayed to perform spinning.

Specifically, the polymer solution is added into a syringe, and the polymer solution is sprayed from the needle toward the collector while applying a voltage of about 1 to 100 kV between the needle at a tip end of the syringe and the collector. At this time, when the voltage exceeds the threshold value, the repulsive force of the charge overcomes the surface tension of the droplets of the polymer solution, and a jet flow with charges is generated. The solvent evaporates while the jet flow is extended in the electric field to form thin fibers, and the fibers are accumulated on the collector, so that a nonwoven fabric is obtained. In the Japanese Industrial Standards (JIS), the nonwoven fabric is defined as "one in which fibers are oriented in one direction or randomly and are bound to each other by entanglement, fusion, or adhesion" (JIS L 0222: 2001).

A suitable applied voltage for electrospinning is 1 kV or more and 100 kV or less. The voltage is more preferably 5 kV or more and 50 kV or less. The voltage is particularly preferably 10 kV or more and 30 kV or less. When the applied voltage is 1 kV or more, a jet flow with charges is likely to be generated, and liquid dripping is unlikely to occur when the HFIP solution of silk fibroin is sprayed from the needle. When the applied voltage is 100 kV or less, the velocity and shape of the jet flow can be easily controlled.

A suitable distance (also referred to as a spinning distance) between the needle and the collector when the electrospinning is performed is 5 cm or more and 50 cm or less. The suitable distance is more preferably 7.5 cm or more and 40 cm or less. The suitable distance is particularly preferably 10 cm or more and 30 cm or less. When the distance between the needle and the collector is 5 cm or more, the solvent is likely to evaporate sufficiently, and liquid dripping is unlikely to occur. When the distance between the needle and the collector is 50 cm or less, a jet flow is likely to be generated.

The rotational speed of the collector is not particularly limited, and is preferably 30 rpm or more and 500 rpm or less, and more preferably 50 rpm or more and 150 rpm or less.

The temperature in the electrospinning method is not particularly limited, and is preferably 0°C or higher and 80°C or lower, more preferably 15°C or higher and 40°C or lower, which is approximate to room temperature (20 to 25°C).

The humidity in the electrospinning method is not particularly limited, and is preferably 20% or more and 80% or less, more preferably 20% or more and 60% or less, and particularly preferably 20% or more and 40% or less. As described above, when the temperature and the humidity are appropriately adjusted, the value of the abs intensity ratio [abs (1650)/abs (1620)] is easily adjusted to be larger than 0.65 and 1.90 or less.

As for the pressure during electrospinning, as long as it is not necessary to adjust the evaporation rate of the solvent in the silk fibroin solution by pressurizing or depressurizing the inside of the electrospinning apparatus, it is preferable to perform the electrospinning at atmospheric pressure (1013.25 hPa on the sea surface) without a need to pressurize or depressurize the inside of the electrospinning apparatus. In addition, it is not necessary to provide a pressurizing/depressurizing mechanism in the electrospinning apparatus, which is economical.

In the present specification, a nonwovens electrospinning system NANON-03 manufactured by MECC Co., Ltd. was used as an apparatus for electrospinning.

The nonwoven fabric containing silk fibers produced by the electrospinning method as described above can be subjected to an insolubilization treatment by contacting (for example, immersing) the nonwoven fabric with alcohol and drying the nonwoven fabric. As a result, the proportion of the β-sheet structures in the nonwoven fabric can be increased, and the value of the abs intensity ratio [abs (1650)/abs (1620)] can be easily adjusted to a range of 0.7 or more and 1.0 or less.

The alcohol is not particularly limited, and examples thereof include water-soluble alcohols such as methanol, ethanol, n-propyl alcohol, and isopropanol. Preferably, ethanol is easily available and easy to handle.

The alcohol may be an alcohol aqueous solution.

Examples of the method of contact with alcohol include known contact methods such as a method in which a nonwoven fabric is immersed in alcohol, a method in which a nonwoven fabric is exposed to vapor of alcohol, and a method in which alcohol is spray-applied to a nonwoven fabric. From the viewpoint of uniform insolubilization treatment in a short period of time, a method of immersing the nonwoven fabric in alcohol is preferred.

The time of contact with the alcohol is not particularly limited, and is generally preferably 10 minutes or more and 5 hours or less, more preferably 10 minutes or more and 3 hours or less, and still more preferably 10 minutes or more and 1 hour or less.

The temperature of contact with the alcohol is not particularly limited, and is preferably 10°C or higher and 50°C or lower, and more preferably 15°C or higher and 40°C or lower. The contact with the alcohol may be performed under a pressurization condition.

The pressurization condition is not particularly limited, and is preferably an atmospheric pressure (1013.25 hPa on the sea surface) or more and twice the atmospheric pressure (2026.5 hPa) or less.

The drying is not particularly limited as long as the insolubilized nonwoven fabric is dried, and examples thereof include constant temperature drying and hot air drying.

When the abs intensity ratio [abs (1650)/abs (1620)] of the nonwoven fabric before contact with alcohol is 1.9 or less, the abs intensity ratio [abs (1650)/abs (1620)] of the nonwoven fabric after contact with alcohol becomes 0.65 or less, and the nonwoven fabric becomes brittle. The abs intensity ratio [abs (1650)/abs (1620)] of the nonwoven fabric obtained by adjusting the humidity during electrospinning before the nonwoven fabric is produced to 50% or more and 70% or less can be 1.90 or more, and the abs intensity ratio [abs (1650)/abs (1620)] of the nonwoven fabric after contact with alcohol can be set to 0.70 or more and 0.80 or less by the contact with alcohol (see [Examples] in the present specification).

The nonwoven fabric includes silk fibers, and may be a nonwoven fabric (silk fiber nonwoven fabric) formed of only silk fibers.

The thickness of the nonwoven fabric containing silk fibers can be controlled to a desired value by appropriately adjusting the amount and concentration of the silk fibroin solution as a raw material. In the case of a nonwoven fabric containing silk fibers obtained by an electrospinning method, the thickness is preferably 1 µm or more and less than 250 µm because the thickness can be easily controlled. The thickness is more preferably 1 µm or more and 200 µm or less, and still more preferably 5 µm or more and 150 µm or less. The thickness is particularly preferably 10 µm or more and 100 µm or less. In addition, in the nonwoven fabric containing silk fibers obtained by the electrospinning method, a support for holding the nonwoven fabric may be further bonded to one side or both sides of the obtained nonwoven fabric depending on the application, and the material of the support is not particularly limited.

A preferred fiber diameter (thickness) (diameter in a circular cross section) of the silk fiber (fiber) constituting the nonwoven fabric is 10 nm or more and 10 µm or less. The fiber diameter is more preferably 20 nm or more and 5 µm or less. The fiber diameter is particularly preferably 30 nm or more and 1 µm or less. Fibers having a fiber diameter of 10 nm or more and 10 µm or less are easily obtained by an electrospinning method.

The above nonwoven fabric is preferably a nonwoven fabric for a blood compatible material.

The present invention also relates to a blood compatible material including a nonwoven fabric for a blood compatible material containing silk fibers.

The blood compatible material is a material used by being brought into contact with blood.

The blood compatible material is not particularly limited, and examples thereof include an artificial blood vessel, a catheter, a blood filter, an anti-adhesion material, a stent, a cell scaffold material, an artificial organ, a blood storage container, and a blood transfusion equipment. The nonwoven fabric containing silk fibers according to one embodiment of the present invention can be particularly suitably used for artificial blood vessels that are always in contact with blood. These blood compatible materials include silk fibers in at least a part thereof, and may include other constituent members. For example, these blood-compatible materials may be a multilayer body in which a nonwoven fabric for a blood material containing silk fibers is applied on a base member (for example, a polymer member containing a synthetic polymer or a biopolymer, or a metal member).

The above nonwoven fabric is preferably a wound dressing.

The nonwoven fabric is preferably an iPS cell scaffold material.

The iPS cells represent induced pluripotent stem cells, and are pluripotent stem cells.

The term "stem cell" refers to a cell having self-replication ability and differentiation ability. Among stem cells, stem cells having self-replication ability and being capable of differentiating from one cell into all cells of endoderm, mesoderm, and ectoderm are referred to as "pluripotent stem cells".

As described above, the method for producing a nonwoven fabric containing silk fibers according to one embodiment of the present invention has been described, and a method for producing a nonwoven fabric containing silk fibers according to a preferred embodiment of the present invention is as follows.

A method for producing a nonwoven fabric containing silk fibers includes: dissolving silk fibroin in a fluorine-based alcohol to obtain a silk fibroin solution; and performing electrospinning using the silk fibroin solution.

In the method for producing a nonwoven fabric containing silk fibers, an abs intensity ratio [abs (1650)/abs (1620)] of the silk fibers, which is a ratio of an intensity of a peak positioned in a vicinity of 1650 cm⁻¹ [abs (1650)] in an infrared absorption spectrum to an intensity of a peak positioned in a vicinity of 1620 cm⁻¹ [abs (1620)] in an infrared absorption spectrum, is larger than 0.65 and 1.90 or less.

Silk fibroin, a fluorine-based alcohol, a silk fibroin solution, electrospinning, an abs intensity ratio [abs (1650)/abs (1620)], and the like are as described above.

The fluorine-based alcohol is preferably a solvent containing 70 mass% or more of 1,1,1,3,3,3-hexafluoro-2-propanol.

The silk fibroin solution is preferably obtained by dissolving silk fibroin in the solvent containing 70 mass% or more of 1,1,1,3,3,3-hexafluoro-2-propanol at a dissolution temperature of 58.6°C or higher and 180°C or lower.

A fluorine-based alcohol, a silk fibroin solution, a solvent containing 70 mass% or more of 1,1,1,3,3,3-hexafluoro-2-propanol, and the like are as described above.

The fluorine-based alcohol is preferably 1,1,1,3,3,3-hexafluoro-2-propanol.

It is preferable to further include a step of bringing the nonwoven fabric containing silk fibers obtained by the electrospinning into contact with an alcohol. The step of contacting with alcohol is as described above.

The alcohol is preferably ethanol or an aqueous solution thereof.

The nonwoven fabric is preferably a wound dressing.

The nonwoven fabric is preferably an iPS cell scaffold material.

The nonwoven fabric is preferably a nonwoven fabric for a blood compatible material (a nonwoven fabric for a blood compatible material containing silk fibers).

Another embodiment of the present invention also relates to a method for producing a blood compatible material, which includes a method for producing a nonwoven fabric for a blood compatible material containing the silk fibers.

### [Examples]

The present invention will be described in more detail by way of Examples, but the present invention is not limited to the following Examples as long as the gist of the present invention is not exceeded.

As a culture method of iPS cells in one embodiment of the present invention, a known culture method was used in which a culture medium (liquid) and a scaffold material were prepared in a plastic petri dish, and iPS cells adhered to the scaffold material and were cultured. The undifferentiation ability of iPS cells after cell culture was evaluated in accordance with known methods by a flow cytometry method and Real-time PCR. An example of a culture method will be described in an Example of the present invention.

### [Production of nonwoven fabric containing silk fibers]

### Preparation of Silk Fibroin HFIP Solution

In a 100 mL stainless steel pressure-resistant container equipped with a thermometer and a stirrer, 0.2 g of silk fibroin and 19.8 g of HFIP were weighed and added at 25°C. Next, the temperature of the inside of the pressure-resistant container was raised to 110°C, and then the mixture was stirred for 2 hours to dissolve silk fibroin, thereby obtaining an HFIP solution containing silk fibroin in a concentration of 1 mass%. From the obtained silk fibroin HFIP solution, 10.0 g of HFIP was distilled off under reduced pressure using an evaporator to obtain 10.0 g of a silk fibroin HFIP solution having a concentration of 2 mass%.

### [Example 1]

### Spinning Based on Electrospinning

Using a nanowovens electrospinning apparatus NANON-03 manufactured by MECC Co., Ltd., spinning was performed on the silk fibroin HFIP solution obtained above. The inner diameter of a nozzle was set to 0.4 mm, an applied voltage was set to 25 kV, and a spinning distance was set to 20 cm. The temperature of the spinning apparatus was maintained at 23 to 24°C, and the humidity was maintained at 20 to 30%. In addition, a drum roll collector was used as a collector, and imitation paper was used on a collector electrode. The rotational speed of the drum collector was 100 rpm. The obtained nonwoven fabric had a thickness of 20 to 50 µm and a fiber diameter of 100 to 700 nm.

### [Comparative Example 1]

### Spinning Based on Electrospinning

Spinning was performed in the same manner as in Example 1 except that the temperature of the spinning apparatus was maintained at 26 to 27°C and the humidity was maintained at 50 to 60%. The obtained nonwoven fabric had a thickness of 20 to 50 µm and a fiber diameter of 100 to 700 nm.

### [Example 2]

A nonwoven fabric produced under the same conditions as in Comparative Example 1 was immersed in ethanol at 23°C for 20 minutes and dried to obtain an insolubilized nonwoven fabric.

### [Example 3]

A nonwoven fabric produced under the same conditions as in Comparative Example 1 was immersed in ethanol at 23°C for 1 hour and dried to obtain an insolubilized nonwoven fabric.

### [Example 4]

A nonwoven fabric produced under the same conditions as in Comparative Example 1 was immersed in ethanol at 23°C for 3 hours and dried to obtain an insolubilized nonwoven fabric.

### [Comparative Example 2]

A nonwoven fabric produced under the same conditions as in Example 1 was immersed in a mixed solution of ethanol and water (ethanol:water = 80:20 in mass ratio) at 23°C for 1 hour and dried to obtain an insolubilized nonwoven fabric.

### [Example 5]

In a 100 mL stainless steel pressure-resistant container equipped with a thermometer and a stirrer, 0.2 g of silk fibroin and 19.8 g of HFIP were weighed and added at 25°C. The silk fibroin was dissolved by stirring the mixture for 2 hours, and thereby, an HFIP solution containing silk fibroin in a concentration of 1 mass% was obtained. From the obtained silk fibroin HFIP solution, 15.0 g of HFIP was distilled off under reduced pressure using an evaporator to obtain 5.0 g of a silk fibroin HFIP solution having a concentration of 4 mass%.

Spinning was performed under the same conditions as in Example 1 except that the silk fibroin HFIP solution was used. The obtained nonwoven fabric had a thickness of 20 to 50 µm and a fiber diameter of 100 to 700 nm.

### <Measurement of Film Thickness of Silk Fiber Nonwoven Fabric>

For the measurement of the film thickness of the silk fiber nonwoven fabric, a micrometer (product name: MDC-25MX, manufactured by Mitutoyo Corporation) was used. In the measurement, three optional points including the center of the nonwoven fabric were measured, and an average value of the thicknesses of the three points was defined as the film thickness.

### <Evaluation of Higher-order Structure of Silk Fiber>

In IR spectrum measurement of the obtained silk fiber nonwoven fabric, the abs intensity ratio [abs (1650)/abs (1620)] was calculated from the peak intensity (abs) in a vicinity of 1650 cm⁻¹ derived from an α-helix and the peak intensity (abs) in a vicinity of 1620 cm⁻¹ derived from a β-sheet structure.

For the IR spectrum measurement, Nicolet iS5 (product name) manufactured by Thermo Fisher SCIENTIFIC was used.

The wavenumber of the peak derived from each high-order structure is described in Non-Patent Literature "Taiyo Yoshioka, Tamako Hata, Katsura Kojima, Yasumoto Nakazawa, and Tsunenori Kameda, Biomater. Sci. Eng., Vol.3, P3207-3214(2017)".

### <Evaluation of Water Resistance of Silk Fiber Nonwoven Fabric>

The silk fiber nonwoven fabrics obtained in Examples and Comparative Examples were immersed in water at room temperature (about 25°C), and the states were visually observed to evaluate the water resistance according to the following criteria.
A: No change in appearance (no dissolution)
B: Partially dissolve
C: Completely dissolve

Table 1 shows the values of the abs intensity ratio [abs (1650)/abs (1620)] of the produced nonwoven fabric and the water resistance evaluation results of the produced nonwoven fabric.

**[Table 1]**

| Silk fiber nonwoven fabric | Ethanol treatment | abs intensity ratio [abs (1650)/abs (1620)] | Water resistance |
|---|---|---|---|
| Example 1 | Untreated | 1.81 | B |
| Example 2 | Immersed for 20 minutes | 0.75 | A |
| Example 3 | Immersed for 1 hour | 0.74 | A |
| Example 4 | Immersed for 3 hours | 0.75 | A |
| Example 5 | Untreated | 1.69 | B |
| Comparative Example 1 | Untreated | 2.15 | c |
| Comparative Example 2 | Immersed in mixed liquid of ethanol and water for 1 hour | 0.65 | - |

As shown in Table 1, in Comparative Example 1 in which the abs intensity ratio [abs (1650)/abs (1620)] was 2.15, the nonwoven fabric was completely dissolved, resulting in poor water resistance. In contrast, in Example 1 in which the abs intensity ratio [abs (1650)/abs (1620)] was 1.81 and Example 5 in which the abs intensity ratio [abs (1650)/abs (1620)] was 1.69, only partial dissolution was observed, and shapes of the nonwoven fabrics tended to be retained easily against water. Furthermore, in Examples 2 to 4 in which the abs intensity ratio [abs (1650)/abs (1620)] was 1.00 or less, dissolution is not confirmed, and shapes of the nonwoven fabrics can be retained against water.

In Comparative Example 2 in which the abs intensity ratio [abs (1650)/abs (1620)] was 0.65, the nonwoven fabric was broken at the time of production or handling, and the water resistance could not be evaluated. In contrast, in Examples 1 to 5 and Comparative Example 1 in which the abs intensity ratio [abs (1650)/abs (1620)] was larger than 0.65, the nonwoven fabrics were not brittle and were not broken.

Each of the silk fiber nonwoven fabrics produced in Examples 1 to 5 and Comparative Examples 1 and 2 was used as a nonwoven fabric for a blood compatible material.

The evaluation of the water resistance of the silk fiber nonwoven fabrics is as shown in Table 1.

### [Pressure Resistance Test of Nonwoven Fabric for Blood-Compatible Material Containing Silk Fiber]

The silk fiber nonwoven fabric produced in Example 3 was used as a nonwoven fabric for a blood compatible material.

Using the silk fiber nonwoven fabric produced in Example 3, a pressure resistance test of the nonwoven fabric required for a blood compatible material such as an artificial blood vessel was performed. In the pressure resistance test, a polyester fiber nonwoven fabric was also tested in the same manner. The polyester fiber nonwoven fabric is a nonwoven fabric produced by using polyethylene terephthalate (model number: NEH-2070, manufactured by Unitika Ltd.) as a raw material and dissolving the polyethylene terephthalate by using HFIP in the same manner as the silk fiber nonwoven fabric to prepare a solution, and then performing production according to the electrospinning method.

A plastic disposable syringe (internal volume: 10 ml) was prepared, and a plastic filter holder (filter inner diameter: 13 mm) was connected to an outlet side of the syringe. The silk fiber nonwoven fabric produced in Example 3 and the polyester fiber nonwoven fabric were cut out into a circular size having a diameter of 13 mm using scissors, and then each nonwoven fabric was sandwiched between filter holders of the syringe. 5 ml of blood (preserved bovine blood, manufactured by Nippon Bio-test Laboratories Inc.) was enclosed in the syringe, and a pressure of 100 mmHg, 200 mmHg, 300 mmHg, 400 mmHg, or 500 mmHg was applied from a plunger of the syringe to evaluate the degree of leakage of blood from the nonwoven fabric. In this test, when no pressure is applied to the nonwoven fabric, the pressure is 0 mmHg.

The results of the evaluation of the pressure resistance of each nonwoven fabric are shown in Table 2.

### [Evaluation of Pressure Resistance of Nonwoven Fabric]

A: No leakage of blood from the nonwoven fabric occurred.
B: Leakage of blood occurred from the nonwoven fabric.

**[Table 2]**

| Types of nonwoven fabric | Pressure conditions | | | | | |
|---|---|---|---|---|---|---|
| | 0 mmHg | 100 mmHg | 200 mmHg | 300 mmHg | 400 mmHg | 500 mmHg |
| Silk fiber nonwoven fabric | A | A | A | A | A | A |
| Polyester fiber nonwoven fabric | A | A | A | B | B | B |

As shown in Table 2, no leakage of blood occurred in the silk nanofiber nonwoven fabric within the range of 0 mmHg to 500 mmHg. In the polyester fiber nonwoven fabrics, leakage of blood did not occur within the range of 0 mmHg to 200 mmHg, but leakage of blood occurred at pressures of 300 mmHg, 400 mmHg, and 500 mmHg. Silk nanofiber nonwoven fabric does not leak blood at each pressure, and has preferred pressure resistance characteristics as a nonwoven fabric for a blood compatible material.

### [Blood Adhesion Test of Nonwoven Fabric for Blood-Compatible Material Containing Silk Fiber]

Using the silk fiber nonwoven fabric produced in Example 3, the blood adhesion of the nonwoven fabric required for a blood compatible material such as an artificial blood vessel was tested. The silk fiber nonwoven fabric and the polyester fiber nonwoven fabric were cut into a 2 cm × 2 cm square using scissors, and then blood (preserved bovine blood, manufactured by Nippon Bio-test Laboratories Inc.) was brought into contact with an entire surface of each nonwoven fabric. Thereafter, the entire surface of each nonwoven fabric was thoroughly washed with distilled water. The appearance of each nonwoven fabric was visually observed and observed with an optical microscope to evaluate the blood adhesion relative to each nonwoven fabric.

The results of the blood adhesion test of each nonwoven fabric are shown in Table 3.

### [Evaluation of Blood Adhesion of Nonwoven Fabric]

A: No adhesion of blood to the nonwoven fabric was observed.
B: Adhesion of blood to the entire surface of the nonwoven fabric was observed.

**[Table 3]**

| Types of nonwoven fabric | Evaluation of blood adhesion |
|---|---|
| Silk fiber nonwoven fabric | A |
| Polyester fiber nonwoven fabric | B |

As shown in Table 3, adhesion of blood to the silk nanofiber nonwoven fabric was not observed. However, adhesion of blood to the polyester fiber nonwoven fabric was observed. Silk nanofiber nonwoven fabric is preferred as nonwoven fabric for a blood compatible material because the nonwoven fabric has a property that blood is less likely to adhere it.

Each of the silk fiber nonwoven fabrics produced in Examples 1 to 5 and Comparative Examples 1 and 2 was used as a wound dressing.

The evaluation of the water resistance of the wound dressing is as shown in Table 1.

### [Evaluation Test of Healing Effect of Wound Dressing]

The silk fiber nonwoven fabric produced in Example 3 was used as a wound dressing.

The wound dressing was tested using rats (Slc: Wistar, male, supply source: Japan SLC, Inc.) as experimental animals. As rats at the start of the test, 16 rats having a body weight of 142 to 154 g and an age of 8 weeks were used. Experimental rats were divided into four groups of a negative control group (CT) for which a wound dressing was not used, a chitin group (CH) for which a chitin wound dressing (manufactured by Nipro Corporation, trade name: Beschitin W) was used, a polyester group (E) for which a polyester wound dressing (manufactured by Unitika Ltd., polyethylene terephthalate, model number: NEH-2070) was used, and a silk group (Si) for which the silk fiber nonwoven fabric produced in Example 3 was used, and four experimental animals of each group were used.

The experimental animals were anesthetized with isoflurane and shaved. A full-thickness defect wound having a diameter of 15 mm was generated using a piercing punch. The wound dressings of the group E and the group Si were wetted with physiological saline and adhered to a wound site. In the group CH, the wound dressing was attached to a wound site according to a product package insert. After attaching the wound dressing, gauze was attached from above the wound dressing, followed by fixing the gauze with a surgical tape, and a net bandage was attached to the body so as to cover the gauze. In the group CT, gauze wetted with physiological saline was directly attached to a wound site and fixed with a surgical tape, and then a net bandage was attached to the body so as to cover the gauze. Elizabethan collars were attached to all the rats after the production of the model animals on the production date of the model animal. After the model animal production day, the Elizabethan collar was replaced once every 2 to 4 days.

After 4 days and 7 days, the wound dressing, gauze, and net bandage were replaced, but when the wound dressing and gauze were not peeled off from the wound site, the wound dressing and gauze were wetted with physiological saline and then peeled off. When the wound dressing was not peeled off even if it was wetted, only the gauze or net bandage attached thereon was replaced without forcibly peeling off the wound dressing.

Regarding all the rats after the production of the model animals, the photographs and the area measurement of the wound sites were performed on the model animal production date (starting time), 4 days later, 7 days later, and 14 days later. The rats were anesthetized with isoflurane inhalation anesthesia.

The wound dressing, gauze, and net bandage were peeled off under anesthesia, and the images of the wound sites were captured with a digital camera.

The rats were anesthetized with isoflurane inhalation anesthesia. The wound dressing, gauze, and net bandage were peeled off under anesthesia, and the shapes of the wound sites were transferred to tracing paper. When the wound dressing or gauze was not peeled off from the wound site, the wound dressing or gauze was wetted with physiological saline and then peeled off. When the wound dressing or gauze was not peeled off even if it was wetted, the shape of the wound site was transferred through the wound dressing or gauze without forcibly peeling off the wound dressing or gauze. Information on the tracing paper was input to a computer using an image scanner. The area was measured using Image J software (US National Institutes of Health).

The wound site area and the proportion (%) of the wound site area are shown in Table 4.

**[Table 4]**

| Animal# | Group | Sex | Wound site area (mm²) | | | | Wound site area (%) | | | |
|---|---|---|---|---|---|---|---|---|---|---|
| | | | Starting time | 4 days later | 7 days later | 14 days later | Starting time | 4 days later | 7 days later | 14 days later |
| CT-01 | Negative control group | male | 187.216 | 110.116 | 66.535 | 18.097 | 100.0 | 58.8 | 35.5 | 9.7 |
| CT-02 | | male | 187.209 | 127.895 | 58.356 | 7.609 | 100.0 | 68.3 | 31.2 | 4.1 |
| CT-03 | | male | 177.772 | 102.025 | 98.779 | 36.368 | 100.0 | 57.4 | 55.6 | 20.5 |
| CT-04 | | male | 151.192 | 135.979 | 93.258 | 21.774 | 100.0 | 89.9 | 61.7 | 14.4 |
| CH-01 | Chitin group | male | 153.009 | 108.540 | 82.276 | 22.545 | 100.0 | 70.9 | 53.8 | 14.7 |
| CH-02 | | male | 155.760 | 129.563 | 77.533 | 35.678 | 100.0 | 83.2 | 49.8 | 22.9 |
| CH-03 | | male | 126.918 | 104.445 | 82.770 | 15.379 | 100.0 | 82.3 | 65.2 | 12.1 |
| CH-04 | | male | 147.565 | 108.810 | 56.047 | 22.167 | 100.0 | 73.7 | 38.0 | 15.0 |
| E-01 | Polyester group | male | 154.381 | 97.018 | 68.937 | 55.686 | 100.0 | 62.8 | 44.7 | 36.1 |
| E-02 | | male | 131.133 | 78.703 | 65.676 | 89.845 | 100.0 | 60.0 | 50.1 | 68.5 |
| E-03 | | male | 145.806 | 38.396 | 25.211 | 24.180 | 100.0 | 26.3 | 17.3 | 16.6 |
| E-04 | | male | 173.822 | 154.252 | 132.093 | 48.476 | 100.0 | 88.7 | 76.0 | 27.9 |
| Si-01 | Silk group | male | 186.153 | 122.732 | 106.155 | 18.700 | 100.0 | 65.9 | 57.0 | 10.0 |
| Si-02 | | male | 170.080 | 71.152 | 75.445 | 19.870 | 100.0 | 41.8 | 44.4 | 11.7 |
| Si-03 | | male | 153.454 | 76.530 | 67.637 | 17.406 | 100.0 | 49.9 | 44.1 | 11.3 |
| Si-04 | | male | 215.082 | 148.147 | 121.394 | 44.494 | 100.0 | 68.9 | 56.4 | 20.7 |
| Average | Negative control group | | 175.847 | 119.004 | 79.232 | 20.962 | 100.0 | 67.7 | 45.1 | 11.9 |
| | Chitin group | | 145.813 | 112.840 | 74.657 | 23.942 | 100.0 | 77.4 | 51.2 | 16.4 |
| | Polyester group | | 151.286 | 92.092 | 72.979 | 54.547 | 100.0 | 60.9 | 48.2 | 36.1 |
| | Silk group | | 181.192 | 104.640 | 92.658 | 25.118 | 100.0 | 57.8 | 51.1 | 13.9 |

After 14 days, a cross sectional of the wound site tissue was observed with a microscope to check the recovery of the skin epithelial tissue.

The evaluation of wound site area and the evaluation of cross section of wound site tissue for the wound dressing were performed as follows, and the healing effect was evaluated. The evaluation results are shown in Table 5.

### [Evaluation of Wound Site Area]

A: The area of the wound site is reduced at an early stage (see 4 days later), and the area of the wound site is reduced 14 days later.
B: The area of the wound site is reduced 14 days later.
C: The area of the wound site is relatively reduced 14 days later.
D: The area of the wound site is relatively large after 14 days.

### [Evaluation of Cross Section of Wound Site Tissue]

A: The epithelial layer is regenerated uniformly.
B: The epithelial layer is partially regenerated.
C: Regeneration of the epithelial layer was hardly observed.

**[Table 5]**

| Group | Number of experimental animals | Evaluation results of healing effect | |
|---|---|---|---|
| | | Evaluation of wound site area | Evaluation of cross section of wound site tissue |
| Negative control (CT) group | 4 | B | B |
| Chitin (CH) group | 4 | C | B |
| Polyester (E) group | 4 | D | C |
| Silk (Si) group | 4 | A | A |

As shown in Table 4, the area in the silk group (group Si) was the smallest in terms of the area (average) of the wound site at an early stage up to 4 days later.

As shown in Tables 4 and 5, in the silk (Si) group, the area (average) of the wound site was small, and in the observation of the cross section of the wound site tissue, the regeneration of the epithelial layer was excellent as compared with the other groups, and the tissue was regenerated neatly.

From the above, it can be seen that the silk group (Si) is superior in healing effect to the other groups.

The silk fiber nonwoven fabrics prepared in Examples 1 to 5 and Comparative Examples 1 and 2 were used as iPS cell scaffolding materials.

The water resistance of the iPS cell scaffold materials was evaluated as shown in Table 1.

### [Culture Test of iPS cells]

Hereinafter, a culture test of the iPS cells will be described in detail, but the present invention is not limited to this example.

As human iPS cells, Cellartis human iPS cell line P11025 (European/North African, male, 33.4 years old), manufactured by Takara Bio Inc., was used. As the culture medium, DEF-CS100 Basal Medium of Cellartis DEF-CS100 Culture System, manufactured by Takara Bio Inc., was used. As the culture substrate (cell scaffold), a culture substrate (cell scaffold), which was obtained by applying the silk fiber nonwoven fabric produced in Example 3 above or DEF-CS COAT-1 (non-silk fiber, manufactured by Takara Bio Inc.) as a control (Comparative Example) to a polystyrene petri dish (flat-bottom cell culture plate, manufactured by TPP), was used. As a cell dissociation solution used for the cell culture and passage, CTSTM TrypLE^{™} Select Enzyme (manufactured by Thermo Fisher Scientific, Cat. No. A1285901). As a cell washing solution, D-PBS (-) (manufactured by Wako, Cat. No. 045-29795, Lot. No. APG7057) was used. As a cell preservation solution, STEM-CELLBANKER (manufactured by ZENOAQ RESOURCE Co., Ltd, Cat. No. CB045) was used.

The iPS cells were cultured on the scaffold of each of the silk fiber nonwoven fabric and the DEF-CS COAT-1 coat until they became confluent, and the iPS cells were observed with an inverted microscope (described as the first passage). Further, iPS cells were passaged to a newly prepared silk fiber nonwoven fabric and DEF-CS COAT-1 coat, and culture was repeated. Finally, when the third passage was completed, two types of flow cytometry and Real-time PCR were performed in order to check undifferentiation of iPS cells.

### 1. Cell Culture

### 1-1 Thawing of Freezed Cells

1). DEF-CS COAT-1 was diluted 20 times with D-PBS (+/+), and added in a culture container to reach 0.1 mL/cm².
2). The diluted DEF-CS COAT-1 was incubated at 37°C for 2 hours or more.
3). A required amount of culture medium, in which DEF-CS GF3 was added to the culture medium so as to be diluted 1,000 times, was prepared.
4). A required amount of culture medium (GF3+) was added in a 15 mL tube.
5). The target cells were taken out from the liquid nitrogen and thawed in a water bath at 37°C.
6). The thawed cells were transferred to the tube prepared in the item 4). To a frozen tube, 1 mL of a culture medium was added, and the thawed cells were added to the tube.
7). The centrifugation was performed at room temperature (25°C) and 300 × g for 1 minute.
8). After the medium is removed by suction from the cells after centrifugation, 1 mL of a culture medium (GF3+) is added and the mixture was suspended by pipetting.
9). DEF-CS COAT-1 was removed from the coated plate in the item 2), and a required amount of culture medium (GF3+) was added in the plate.
10). The cells were inoculated to the plate of the item 9) in an amount of about 3.0 × 10⁵ cells/cm².
11). The cells were cultured in the presence of 5% CO₂ at 37°C.
12). On the next day, medium replacement is performed with a culture medium, and then, medium replacement is performed every other day.

### 1-2 Passage Culture

### <Control> (Comparative Example)

1). DEF-CS COAT-1 was diluted 20 times with D-PBS (+/+), and added in a culture container to reach 0.1 mL/cm².
2). The diluted DEF-CS COAT-1 was incubated at 37°C for 2 hours or more.
3). 70 to 80% confluent cells were washed with 1 mL/well of D-PBS (-).
4). D-PBS (-) is aspirated, TrypLE^{™} Select was added in an amount of 76 µL/well to reach 20 µL/cm², followed by standing at 37°C for 5 minutes, and the adhesion between the cells was dissociated.
5). The culture medium (GF3+) was added in an amount of 500 µL/well, and the mixture was suspended by pipetting to obtain single cells.
6). Centrifugation was performed at room temperature (25°C) and 200 × g for 4 minutes.
7). The supernatant was aspirated, and the cell pellets were suspended in a culture medium (GF3+) of 500 µL/well.
8). The number of cells was counted.
9). DEF-CS COAT-1 was aspirated from the cell container coated in the item 2), and a culture medium (GF3+) was added to reach 0.17 mL/cm².
10). The cells were inoculated to the plate of the item 9) to reach an amount of about 0.6 × 10⁵ to 1.4 × 10⁵ cells/cm².
11). The cells were cultured in a CO₂ incubator at 37°C.
12). On the next day, medium replacement was performed with a culture medium, and then, medium replacement was performed every two days. When the number of cells increased and the medium started to become yellow on the next day after the culture medium replacement, medium replacement was performed every day.

### <Use of Silk Fiber Nonwoven Fabric> (Example)

1). Each of the silk fiber nonwoven fabrics was cut into a circle having a diameter of about 2.1 cm.
2). The circular silk fiber nonwoven fabrics were immersed in 70% ethanol and sterilized.
3). The silk fiber nonwoven fabrics were air-dried and then washed several times with D-PBS (-).
4). The silk fiber nonwoven fabrics were placed in wells of a 12-hole plate.
5). A culture medium (GF3+) was added.
6). Incubation was performed at 37°C for 2 hours or more.
7). 70 to 80% confluent cells were washed with 1 mL/well of D-PBS (-).
8). D-PBS (-) was aspirated, TrypLE^{™} Select was added in an amount of 76 µL/well to reach 20 µL/cm², followed by standing at 37°C for 5 minutes, and the adhesion between the cells was dissociated.
9). The culture medium (GF3+) was added in an amount of 500 µL/well, and the mixture was suspended by pipetting to obtain single cells.
10). Centrifugation was performed at room temperature (25°C) and 200 × g for 4 minutes.
11). The supernatant was aspirated, and the cell pellets were suspended in a culture medium (GF3+) of 500 µL/well.
12). The number of cells was counted.
13). The cells were inoculated to the plate of the item 5) to reach an amount of about 2.4 × 10⁴ to 8.3 × 10⁴ cells/cm².
14). The cells were cultured in a CO₂ incubator at 37°C.
15). On the next day, medium replacement was performed with a culture medium, and then, medium replacement was performed every two days. When the number of cells increased and the medium started to become yellow on the next day after the culture medium replacement, medium replacement was performed every day.

### [Results of Culture Test of iPS Cells]

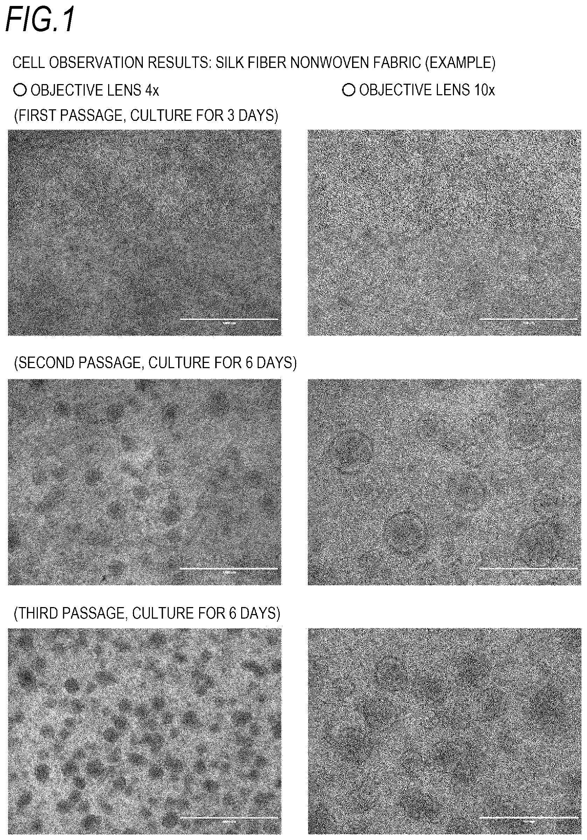

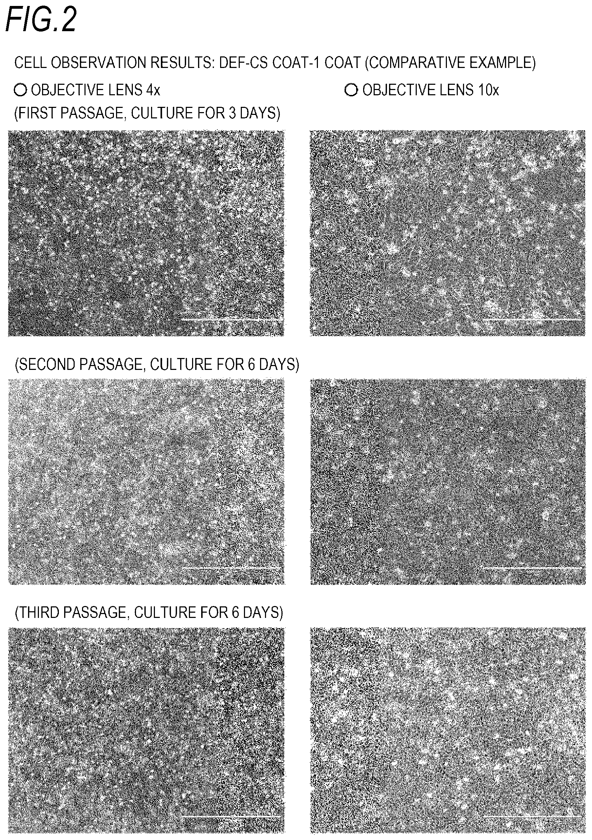

Microscope observation was performed after respective cultures of the first passage, the second passage, and the third passage, and states at the time of iPS cell proliferation in Examples and Comparative Examples were compared. The microscopic observation photographs are shown in FIGs. 1 and 2.

In the Example in which the silk fiber nonwoven fabric was used as the scaffold material, a mass such as black spots was confirmed in the microphotograph after the culture. This suggests that iPS cells proliferate while forming a mass and becoming round upward (three-dimensionally).

In contrast, in the Comparative Example in which DEF-CS COAT-1 was used as a scaffold material without using the silk fiber nonwoven fabric, the mass as described above was not recognized, and it was suggested that iPS cells increased while spreading on a plane not three-dimensionally but two-dimensionally.

Under the culture conditions of this time, the growth process of iPS cells which is generally predicted is planar growth relative to the scaffold material, and three-dimensional growth has a high level of technical difficulty.

The results of cell culture are shown in the following table.

**[Table 6]**

| (DEF-CS COAT-1 coat) | | | | | | |
|---|---|---|---|---|---|---|
| Passage number | Culture days (day) | Inoculating number (× 10⁵ cells) | Total number of cells (× 10⁵ cells) | Survival rate (%) (at the time of recovering) | Proliferation rate (times) | Doubling time (hr) |
| First passage | 3 | 0.5 | 2.25 | 96.2 | 4.5 | 33.2 |
| Second passage | 6 | 0.21 | 9.12 | 100 | 43.4 | 26.5 |
| Third passage | 6 | 0.21 | 9.62 | 99.4 | 45.8 | 26.1 |

| | | | | | | |
|---|---|---|---|---|---|---|
| ^{∗}Calculation formula of doubling time: Doubling time (hr) = ln2/ln (proliferation rate) × culture days (day) × 24 (hr) | | | | | | |

**[Table 7]**

| (Nonwoven fabric containing silk fibers) | | | | | | |
|---|---|---|---|---|---|---|
| Passage number | Culture days (day) | Inoculating number (× 10⁵ cells) | Total number of cells (× 10⁵ cells) | Survival rate (%) (at the time of recovery) | Proliferation rate (times) | Doubling time (hr) |
| First passage | 3 | 3 | 0.86 | 86.4 | 0.3 | - |
| Second passage | 6 | 0.86 | 5.92 | 100 | 6.9 | 51.6 |
| Third passage | 6 | 0.86 | 14.41 | 98.9 | 16.8 | 35.4 |

The above tables describe the total number of cells indicating how much the iPS cells have proliferated after culture by first inoculating the iPS cells, the survival rate indicating the proportion of live iPS cells after excluding dead cells in the total cells, and the like.

From the above tables, it can be seen that the total number of cells of the iPS cells of this time was increased, and the proportion of dead cells was not high even on the silk fiber nonwoven fabric scaffold, similarly to the COAT-1 scaffold as the control.

In the iPS cell culture, it is necessary to confirm that the number of cells is increased and that the undifferentiation ability is also maintained.

In order to confirm the undifferentiation of the iPS cells cultured on the silk fiber nonwoven fabric, the expressions of SSEA-4 and TRA-1 to 60, which were undifferentiation markers, were separately tested by flow cytometry.

Specifically, in order to evaluate the undifferentiation ability of the iPS cells, analysis was performed using a flow cytometer (FACS Aria, manufactured by BD Biosciences Inc.). The evaluation results are shown below.

**[Table 8]**

| Scaffold material | Total number of cells | SSEA-4 positive cells | TRA-1-60 positive cells |
|---|---|---|---|
| Silk fiber | 9852 | 9795 (99.4%) | 8967 (91.0%) |
| DEF-CS COAT-1 | 9872 | 9841 (99.7%) | 9581 (97.1%) |

As a result, it was confirmed that the iPS cells cultured on the silk fiber showed the same level of positive results as those of the control test in SSEA-4 and TRA-1-60, which were undifferentiation markers on the surfaces of the iPS cells, and it was found that the undifferentiation ability was maintained.

In addition, in order to confirm undifferentiation of the iPS cells cultured on the silk fiber nonwoven fabric, expressions of Oct3/4 and NANOG were separately tested by Real-Time-PCR.

Specifically, in order to evaluate the undifferentiation ability of the iPS cells, the expression levels of Oct3/4 and NANOG, which were undifferentiation markers, were evaluated using Real-time PCR (Step One Plus real-time PCR system, manufactured by Thermo Fisher Scientific Inc.). The evaluation results are shown below.

**[Table 9]**

| Scaffold material | Total number of RNAs (µg) | GAPDH (internal standard gene) | Oct 3/4 | NANOG | [Oct3/4]/GAPDH | NANOG/GAPDH |
|---|---|---|---|---|---|---|
| Silk fiber nonwoven fibric | 9.4 | 1815741 | 652936 | 33538 | 0.36 | 0.018 |
| DEF-CS COAT-1 | 9.2 | 1436038 | 648638 | 24136 | 0.45 | 0.017 |

As a result, it was confirmed that the iPS cells cultured on the silk fiber nonwoven fabric exhibited the same expression level as that of the control test, and it was found that the undifferentiated ability was maintained.

Specifically, the total numbers of RNAs of the iPS cells cultured on the silk fiber nonwoven fabric and the control were aligned and subjected to gene analysis, and as a result, it was found that the expression level proportion of the control was high in terms of Oct3/4 and the expression level proportions thereof are at the same level in terms of Oct3/4, and the undifferentiation was maintained.

### INDUSTRIAL APPLICABILITY

According to an aspect of the present invention, it is possible to provide a nonwoven fabric containing silk fibers, which has high water resistance and high toughness, and a method for producing the nonwoven fabric.

According to another aspect of the present invention, it is possible to provide a nonwoven fabric for a blood-compatible material and a blood-compatible material which have high water resistance and high toughness and can prevent leakage of blood and adhesion of blood, a method for producing the nonwoven fabric for the blood-compatible material, and a method for producing the blood-compatible material.

According to still another aspect of the present invention, it is possible to provide a wound dressing that has high water resistance, high toughness, and excellent healing effects, and a method for producing the wound dressing.

According to yet still another aspect of the present invention, it is possible to provide an iPS cell scaffold material that has high water resistance and high toughness and enables iPS cells to form a three-dimensional tissue structure during proliferation, and a method for producing the iPS cell scaffold material.

Although the present invention are described in detail with reference to the specific embodiment, it will be apparent to those skilled in the art that various changes and modifications can be made without departing from the spirit and scope of the present invention.

The present application is based on Japanese Patent Application No. 2019-163170 filed on September 6, 2019, Japanese Patent Application No. 2019-163172 filed on September 6, 2019, Japanese Patent Application No. 2019-185461 filed on October 8, 2019, and Japanese Patent Application No. 2019-185462 filed on October 8, 2019.

## Claims

1. A nonwoven fabric containing silk fibers, wherein an abs intensity ratio [abs (1650)/abs (1620)], which is a ratio of an intensity of a peak positioned in a vicinity of 1650 cm⁻¹ [abs (1650)] in an infrared absorption spectrum to an intensity of a peak positioned in a vicinity of 1620 cm⁻¹ [abs (1620)] in an infrared absorption spectrum, is larger than 0.65 and 1.90 or less.

2. The nonwoven fabric containing silk fibers according to claim 1, wherein the abs intensity ratio [abs (1650)/abs (1620)] is 0.70 or more and 1.00 or less.

3. The nonwoven fabric containing silk fibers according to claim 1 or 2, wherein the nonwoven fabric is a nonwoven fabric for a blood compatible material.

4. A blood compatible material comprising the nonwoven fabric containing silk fibers according to claim 3.

5. The blood compatible material according to claim 4, wherein the blood compatible material is an artificial blood vessel, a catheter, a blood filter, an anti-adhesion material, a stent, a cell scaffold material, an artificial organ, a blood storage container, or a blood transfusion equipment.

6. The nonwoven fabric containing silk fibers according to claim 1 or 2, wherein the nonwoven fabric is a wound dressing.

7. The nonwoven fabric containing silk fibers according to claim 1 or 2, wherein the nonwoven fabric is an iPS cell scaffold material.

8. A method for producing a nonwoven fabric containing silk fibers comprising:
dissolving silk fibroin in a fluorine-based alcohol to obtain a silk fibroin solution; and
performing electrospinning using the silk fibroin solution,
wherein an abs intensity ratio [abs (1650)/abs (1620)] of the silk fibers, which is a ratio of an intensity of a peak positioned in a vicinity of 1650 cm⁻¹ [abs (1650)] in an infrared absorption spectrum to an intensity of a peak positioned in a vicinity of 1620 cm⁻¹ [abs (1620)] in an infrared absorption spectrum, is larger than 0.65 and 1.90 or less.

9. The method for producing a nonwoven fabric containing silk fibers according to claim 8, wherein the fluorine-based alcohol is a solvent containing 70 mass% or more of 1,1,1,3,3,3-hexafluoro-2-propanol.

10. The method for producing a nonwoven fabric containing silk fibers according to claim 8 or 9, wherein the silk fibroin solution is obtained by dissolving silk fibroin in the solvent containing 70 mass% or more of 1,1,1,3,3,3-hexafluoro-2-propanol at a dissolution temperature of 58.6°C or higher and 180°C or lower.

11. The method for producing a nonwoven fabric containing silk fibers according to any one of claims 8 to 10, wherein the fluorine-based alcohol is 1,1,1,3,3,3-hexafluoro-2-propanol.

12. The method for producing a nonwoven fabric containing silk fibers according to any one of claims 8 to 11, further comprising a step of bringing the nonwoven fabric containing silk fibers obtained by electrospinning into contact with an alcohol.

13. The method for producing a nonwoven fabric containing silk fibers according to claim 12, wherein the alcohol is ethanol or an aqueous solution thereof.

14. The method for producing a nonwoven fabric containing silk fibers according to any one of claims 8 to 13, wherein the nonwoven fabric is a wound dressing.

15. The method for producing a nonwoven fabric containing silk fibers according to any one of claims 8 to 13, wherein the nonwoven fabric is an iPS cell scaffold material.

16. The method for producing a nonwoven fabric containing silk fibers according to any one of claims 8 to 13, wherein the nonwoven fabric is a nonwoven fabric for a blood compatible material.

17. A method for producing a blood compatible material, comprising the method for producing a nonwoven fabric containing silk fibers according to claim 16.
